# FASCICULE DE BREVET EUROPEEN

(11) **EP 2 373 263 B1**
(45) Date de publication et mention de la délivrance du brevet: **28.01.2015**
(21) Numéro de dépôt: 09805797.9
(22) Date de dépôt: 28.12.2009
(51) Int. Cl.: A61F 2/40

(54) **IMPLANT GLENOIDIEN**
GLENOID-IMPLANTAT
GLENOID IMPLANT

(30) Priorité: 29.12.2008 FR 0859102
(43) Date de publication de la demande: 12.10.2011
(73) Titulaire: Capon, Didier, 44880 Sautron (FR); Gonzalvez, Martin, 21000 Dijon (FR); Handelberg, Franck, 1652 Alsemberg / Beersel (BE); Isidro, Albert, 08914 Badalona Barcelone (ES); Laques, Damien, 43700 Pujols (FR); De La Selle, Hugues, 71100 Chalon Sur Saone (FR); Vedel, François, 13090 Aix En Provence (FR); Aston Medical Developments Ltd, Londres NW1 1JD (GB)
(72) Inventeur: ALEPEE, Christophe, F-69003 Lyon (FR)
(74) Mandataire: Thivillier, Patrick
(86) Numéro de dépôt international: PCT/FR2009/052711
(87) Numéro de publication internationale: WO 2010/076534

(56) Documents cités:
- EP-A- 0 581 667
- EP-A- 1 402 853
- EP-A2- 0 339 530
- WO-A-2006/086606
- US-A- 5 489 310
- US-A1- 2007 038 302
- US-A1- 2007 260 321

## Description

L'invention se rattache au secteur technique des implants orthopédiques.

Plus particulièrement, l'invention concerne le domaine de l'arthroplastie de l'articulation de l'épaule, notamment le traitement de l'omarthrose centrée.

D'une manière parfaitement connue pour un homme du métier, afin de compenser l'usure d'une glène présentant une arthrose, l'opérateur implante une prothèse glénoïdienne.

Pour l'essentiel, ce type de prothèse comprend, généralement, soit une cupule cimentée en polyéthylène, soit une embase non cimentée destinée à recevoir une cupule en polyéthylène sur laquelle vient en appui, avec capacité d'articulation, une tête humérale prothétique.

Il est apparu que ce type de prothèse présente des inconvénients étant donné qu'il a tendance à latéraliser l'articulation, ce qui a pour conséquence de modifier les centres de rotation de l'épaule et d'entraîner un couple d'arrachement entre le point d'implication de l'effort créé par la tête humérale et sa fixation sur la glène. Il en résulte un bras de levier constitué par l'épaisseur de la cupule en polyéthylène qui est de l'ordre de 6 mm lorsqu'elle est cimentée et de 8 à 9 mm lorsque la cupule est associée, comme indiqué, à une embase. Ce bras de levier forme un couple d'arrachement non négligeable susceptible d'entraîner des descellements prématurés.

Or, des études récentes de biomécanique ont démontré que le centre instantané de rotation se situe dans une surface de l'ordre de 5 à 8 mm de diamètre, et non plus suivant une translation suivie d'une rotation de l'articulation selon les publications antérieures et comme il était généralement admis par les praticiens.

A partir de cette constatation, le problème que se propose de résoudre l'invention est de simplement remplacer le cartilage, sans en modifier l'architecture biomécanique. Un autre problème que se propose de résoudre l'invention est d'assurer la fixation de la cupule dans l'os spongieux de la glène.

Pour résoudre ces problèmes, il a été conçu et mis au point un implant glénoïdien comprenant une cupule destinée à coopérer avec une tête humérale prothétique et conforme aux caractéristiques de la revendication 1.

La cupule et l'embase présentent des formes complémentaires d'accouplement. La cupule présente un chambrage coopérant avec une collerette de l'embase.

Compte tenu des efforts de cisaillement de la tête humérale sur la cupule, le pion présente des entailles opposées anti-rotatoires, et une fente transversale permettant son encastrement dans le spongieux de la glène

L'invention est exposée ci-après plus en détail à l'aide des figures des dessins annexés dans lesquels :
- la figure 1 est une vue à caractère schématique d'une omoplate dont la cavité anatomique glénoïdienne est équipée d'un implant selon l'invention ;
- les figures 2 et 3 sont des vues en perspective avant montage de la cupule sur l'embase ;

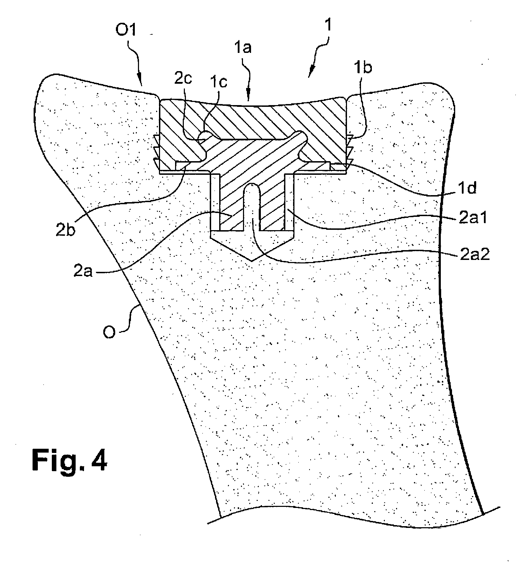

- la figure 4 est une vue en coupe montrant la fixation d'un implant glénoïdien, selon l'invention dans un logement de la cavité anatomique glénoïdienne.

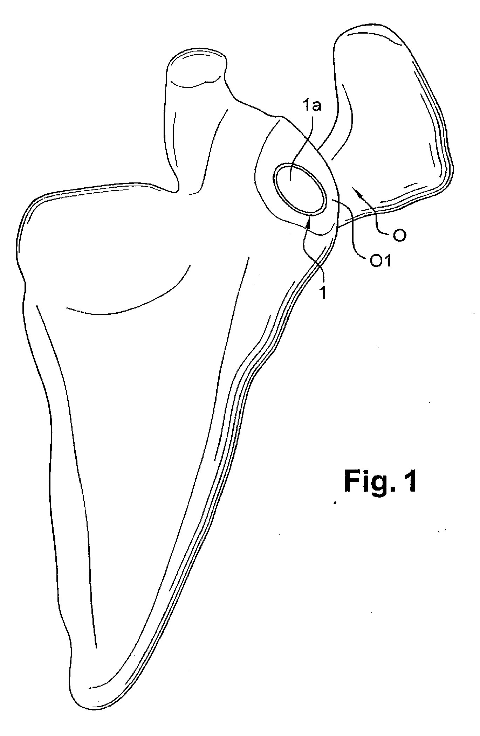

L'omoplate, illustrée figure 1, est désignée par (O), tandis que la cavité anatomique glénoïdienne, destinée à recevoir l'implant, est désignée par (O1).

Selon une caractéristique à la base de l'invention, l'implant glénoïdien est conformé pour être encastré dans la cavité glénoïdienne avec, pour objectif, de simplement remplacer le cartilage, sans en modifier l'architecture biomécanique.

L'implant présente une cupule en polyéthylène (1) présentant une surface d'appui et de glissement concave (1a) pour l'articulation de la tête humérale prothétique (non représentée).

Selon l'invention, la cupule (1) présente des agencements de positionnement et de fixation en vue de son encastrement dans un logement de forme complémentaire formé dans l'os spongieux de la cavité glénoïdienne (O1). Après fixation, la surface d'appui et de glissement (1a) de la cupule (1), qui est de forme générale circulaire, est congruente à la cavité anatomique de la glène.

Autrement dit, la cupule (1) s'intègre dans la continuité de la cavité anatomique.

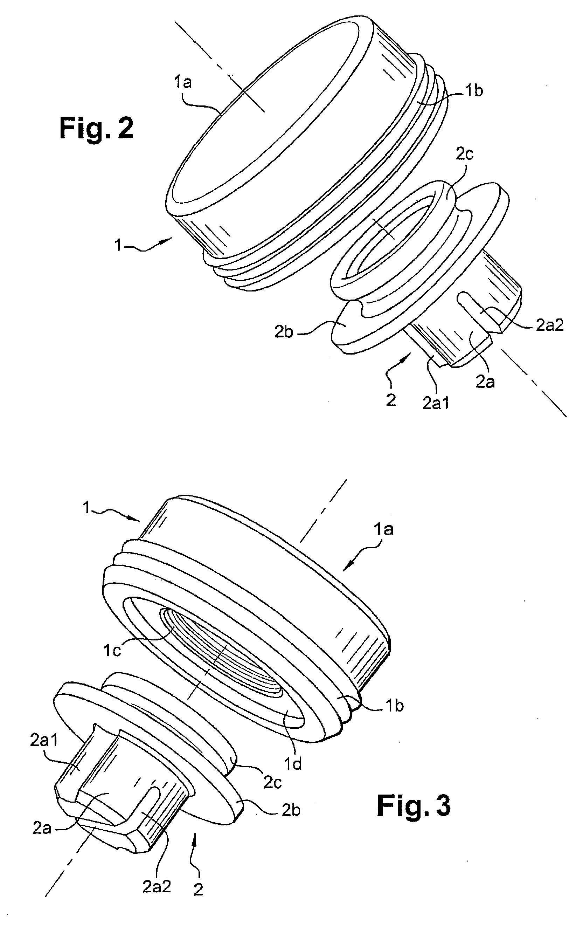

Comme le montrent notamment les figures 2 et 3, la cupule présente, en débordement de sa périphérique, des lamelles déformables de stabilisation (1b) coopérant avec l'os spongieux. Avantageusement, la cupule (1) est accouplée à une embase (2) présentant un pion d'ancrage (2a) dans un logement formé dans l'épaisseur de la cavité glénoïdienne recevant la cupule (1). La cupule (1) et l'embase (2) présentent des formes complémentaires d'accouplement (2c), Par exemple, l'embase (2) présente une collerette d'appui circulaire (2b) formée coaxialement au pion (2a), laquelle présente en débordement les formes de clipage (2c) coopérant, sous une forte pression, avec des formes complémentaires (1c) de la cupule (1).

A noter également que la cupule (1) présente un chambrage (1d) dans lequel est engagée la collerette d'appui (2b) de l'embase (2).

Pour créer un effet antirotatoire sous les efforts de cisaillement de la tête humérale sur la cupule (1), le pion (2a) présente des entailles (2a1) diamétralement opposées, par exemple. A noter que la face d'appui de la collerette (2b) de l'embase (2) et la surface externe du pion (2a) peuvent recevoir un revêtement bioconducteur.

Avantageusement, le pion (2a) présente également une ou deux entailles transversales (2a2) permettant son encastrement dans le spongieux de la glène, en faisant ainsi office de blocage rotatoire et en augmentant la surface de réhabitation de la glène.

Les avantages ressortent bien de la description, en particulier on souligne et on rappelle que l'implant de resurfacage selon les caractéristiques de l'invention, remplace simplement le cartilage, sans en modifier l'architecture biomécanique, en tenant compte du fait que le centre de rotation se situe dans une surface de l'ordre de 5 à 8 mm de diamètre.

## Revendications

1. Implant glénoïdien comprenant une cupule (1) destinée à coopérer avec une tête humérale prothétique, **caractérisé en ce que** la cupule (1) est de forme générale circulaire et présente, en débordement de sa périphérie, des lamelles déformables de stabilisation (1b) coopérant avec le spongieux, ladite cupule est accouplée à une embase (2) présentant un pion d'ancrage (2a) dans la cavité anatomique glénoïdienne, la cupule (1) et l'embase (2) sont encastrées dans ladite cavité, de sorte que la surface d'appui et de glissement (1a) de ladite cupule s'intègre dans la continuité de ladite cavité anatomique, pour obtenir une congruence parfaite avec la tête humérale.

2. Implant selon la revendication 1 2, **caractérisé en ce que** la cupule (1) et l'embase (2) présentent des formes complémentaires d'accouplement (1c et (2c)

3. Implant selon l'une des revendications 1 à 2, **caractérisé en ce que** la cupule (1) présente un chambrage (Id) coopérant avec une collerette (2b) de l'embase.

4. Implant selon l'une des revendications 1 à 3, **caractérisé en ce que** le pion (2a) présente des entailles opposées anti-rotatoires (2a1).

5. Implant selon l'une quelconque des revendications 1 à 4 **caractérisé en ce que** le pion (2a) présente au moins une entaille transversale (2a2).

## Patentansprüche

1. Glenoidimplantat, umfassend einen Becher (1), der dazu bestimmt ist, mit einem prothetischen Humeruskopf zusammenzuwirken, **dadurch gekennzeichnet, dass** der Becher (1) allgemein kreisförmig ist und an seinem Umfang hinausragend verformbare Stabilisierungslamellen (1b) aufweist, die mit der Spongiosa zusammenwirken, wobei der Becher mit einem Sockel (2), welcher einen Stift zur Verankerung (2a) in der anatomischen Schultergelenkpfanne aufweist, gekuppelt ist, der Becher (1) und der Sockel (2) in die Pfanne eingebettet sind, so dass die Auflage- und Gleitfläche (1a) des Bechers sich in den stetigen Verlauf der anatomische Pfanne integriert, um eine perfekte Kongruenz mit dem Humeruskopf zu erhalten.

2. Implantat nach Anspruch 1, **dadurch gekennzeichnet, dass** der Becher (1) und der Sockel (2) ergänzende Kupplungsformen (1c) und (2c) aufweisen.

3. Implantat nach einem der Ansprüche 1 bis 2, **dadurch gekennzeichnet, dass** der Becher (1) eine Einsenkung (1d) aufweist, die mit einem Kragen (2b) des Sockels zusammenwirkt.

4. Implantat nach einem der Ansprüche 1 bis 3, **dadurch gekennzeichnet, dass** der Stift (2a) gegenüberliegende Drehsicherungseinkerbungen (2a1) aufweist.

5. Implantat nach einem der Ansprüche 1 bis 4, **dadurch gekennzeichnet, dass** der Stift (2a) wenigstens eine Quereinkerbung (2a2) aufweist.

## Claims

1. Glenoid implant comprising a cup (1) intended to engage a prosthetic humeral head, wherein the cup (1) is generally circular and has, on the overlap of its periphery, deformable stabilization lamellae (1b) that interact with the cancellous bone, the said cup is connected to a baseplate (2) that has an anchoring lug (2a) in the anatomical glenoid cavity, the cup (1) and baseplate (2) are embedded in the said cavity, such that the load-bearing and sliding surface (1a) of the said cup is integrated into the continuity of the said anatomical cavity, in order to be perfectly congruent with the humeral head.

2. Implant according to claim 1 2, wherein the cup (1) and the baseplate (2) have complementary attachment shapes (1c) and (2c).

3. Implant according to claims 1 to 2, wherein the cup (1) has a recess (1d) interacting with a flange (2b) of the baseplate.

4. Implant according to one of the claims 1 to 3, wherein the lug (2a) has opposite anti-rotation notches (2a1).

5. Implant according to any one of the claims 1 to 4, wherein the lug (2a) has at least one transverse notch (2a2).
